# EUROPEAN PATENT SPECIFICATION

(11) **EP 2 074 961 B1**
(45) Date of publication and mention of the grant of the patent: **07.11.2012**
(21) Application number: 08022005.6
(22) Date of filing: 18.12.2008
(51) Int. Cl.: A61F 2/16

(54) **Intraocular lens injection instrument**
Injektionsinstrument für eine Intraokularlinse
Instrument d'injection de lentille intraoculaire

(30) Priority: 29.12.2007 JP 2007341515
(43) Date of publication of application: 01.07.2009
(73) Proprietor: Nidek Co., Ltd., Gamagori-shi Aichi 443-0038 (JP)
(72) Inventor: Sunada, Chikara, Gamagori-shi Aichi 443-0038 (JP); Nagasaka, Shinji, Gamagori-shi Aichi 443-0038 (JP)
(74) Representative: Hofer, Dorothea

(56) References cited:
- EP-A- 1 360 945
- WO-A-99/62436
- WO-A-2005/070341
- WO-A-2005/074838
- US-A- 4 699 140
- US-B1- 6 447 520

## Description

### BACKGROUND OF THE INVENTION

### 1. Field of the Invention

The present invention relates to an intraocular lens injection instrument for injecting an intraocular lens in an eye.

### 2. Description of Related Art

As one of operative treatments for cataract, heretofore, there has generally been used a method in which a lens is removed from a patient's eye and then a foldable, soft intraocular lens (IOL) is injected in place of the lens. The IOL includes a foldable optic part and support parts each having an open loop shape for supporting this optic part in the eye. For injection of such foldable IOL, an IOL injection instrument called an injector is used to inject the IOL in a folded state into the eye through an incision. Thus, the incision has only to be formed in advance with a minimum diameter in the eye. This injector is configured to advance the IOL set therein by a push rod called a plunger while the IOL is folded into a smaller size, and push out the IOL through a front end of the injector. In a normal state, the support parts of the IOL are located near a plane on which the optic part is placed. In this state, a front end of the plunger may touch a rear one of the support parts of the IOL during injection and push out the IOL with the rear support part being entangled, resulting in a damage of the support part. To prevent the plunger from damaging the rear support part, there is proposed an IOL injection instrument provided with a mechanism for placing the front end of the plunger in advance between the optic part and the support part so that the rear support part is put on the plunger (EP 1360945 A1).

### SUMMARY OF THE INVENTION

However, in the IOL injection instrument disclosed in EP '945, the rear support part is put on the plunger and thus stress is constantly applied to this support part. If this state continues for a long time, the support part is likely to be deformed. To avoid such defect, this instrument is arranged to place the optic part of the IOL in another position (a different position from a movement axis) during storage and move the IOL onto the movement axis prior to injection of the IOL. Therefore, such IOL injection instrument as disclosed in EP '945 needs an operation of moving the IOL to a predetermined position prior to injection of the IOL. An instrument including a mechanism of moving the IOL results in a complicated structure and an increase in the number of parts.

The present invention has an object to overcome the above problems and to provide an intraocular lens injection instrument having a simple structure capable of restraining damage to a support part due to a push rod (a plunger) and appropriately pushing out an intraocular lens by a simple operation.

(1) The above object is attained by an intraocular lens injection instrument according to claim 1; specifically, an intraocular lens injection instrument comprising: an injection part for injecting an intraocular lens (IOL) into an eye through an incision to be formed in the eye, the IOL having a deformable optic part and a pair of support parts each having a loop shape extending from the optic part, and the injection part having an internal shape that allows the IOL to be folded during movement through the injection part; a setting part connected to a rear end of the injection part and configured to set therein the IOL in a state with no stress applied; a main unit of a cylindrical configuration having the injection part and the setting part at a front end; and a push-out member having a push rod provided to be movable forward and backward in the main unit in order to push the IOL along a movement axis out of a front end of the injection part, characterized in that the IOL injection instrument further comprises: a guide member provided in a rear of the setting part and arranged to guide forward and backward movement of the push rod, the guide member being configured to bend the push rod positioned on an axis different from the movement axis and then guide the push rod to move along the movement axis, and also configured to prevent a rear one of the support parts from becoming deformed, and the push rod has adequate flexibility to be deformed along the guide member.
(2) In the intraocular lens injection instrument (1), preferably, the guide member is a tubular member having an entrance for receiving the push rod and an exit for feeding the push rod, the exit being located on the movement axis.
(3) In the intraocular lens injection instrument (1) or (2), preferably, the guide member is formed with a guide groove, and the push rod is provided with a protrusion engageable with the groove.
(4) In the intraocular lens injection instrument (2), preferably, the entrance of the guide member is provided below a setting surface of the setting part.
(5) In the intraocular lens injection instrument (1), preferably, the guide member is formed with a guide groove and the push rod is provided with a protrusion engageable with the groove, and the setting part and the injection part are provided with a groove continuous from the setting part to the injection part and engageable with the protrusion of the push rod.

According to the present invention, it is possible by a simple structure to restrain damage to a support part due to a push rod (a plunger) and appropriately push out an intraocular lens by a simple operation.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

### In the drawings,

Figs. 1A and 1B are external views of an intraocular lens (IOL);
Figs. 2A and 2B are schematic structural views of an IOL injection instrument in a preferred embodiment;
Figs. 3A and 3B are views to explain a setting part;
Fig. 4 is a view to explain centering; and
Fig. 5 is a cross sectional view of the setting part in which a distal end portion contacts is in contact with an optic part.

### DETAILED DESCRIPTION OF THE PREFERRED EMBODIMENTS

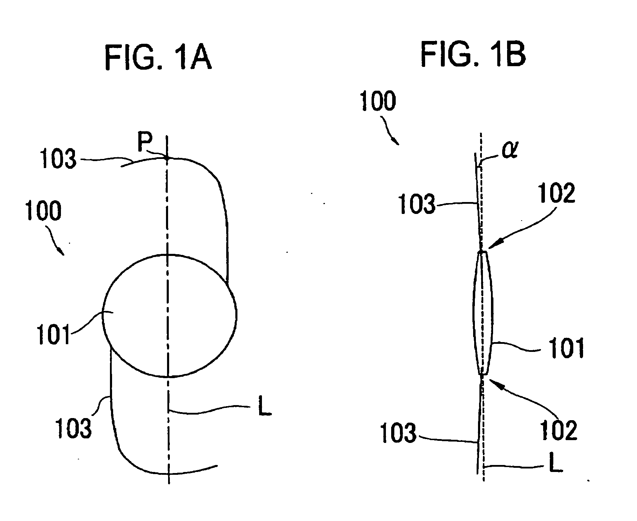

A detailed description of a preferred embodiment of the present invention will now be given referring to the accompanying drawings. Figs. 1 are views showing a configuration of an intraocular lens (IOL) 100 set in advance in an IOL injection instrument in the present embodiment. As shown in the figure, the IOL 100 includes an optic part 101 having predetermined refractive power and a pair of support parts 103 for fixedly supporting the optic part 101 in a patient's eye. The optic part 101 is made of a soft foldable material made of hydrophilic or hydrophobic acrylic resin. The optic part 101 is provided with an edge 102 having a predetermined thickness. The support parts 103 are respectively joined to this edge 102. In a push-out operation of the IOL 100 which will be mentioned later, the edge 102 is to be pushed by a front end portion of a push rod. Each support part 103 has an open loop shape whose one end (a proximal end) is joined to the optic part (the edge 102) and the other end (a distal end) is a free end. Each support part 103 is made of resin such as polymethylmetacrylate (PMMA) and polypropylene. As shown in Fig. 1B, each support part 103 is attached obliquely at a predetermined angle α (e.g., in the order of 0 to 10 degrees) with respect to a front side of the optic part 101.

An axis L in Fig. 1A is a movement axis (a push-out axis) of the IOL 100 to be pushed out. Along this axis L, the IOL 100 will be pushed out. Accordingly, a point P in Fig. 1A will be an intersection point in projection of the push rod 6 and the support part 103 (which will be placed on a rear side when the IOL is loaded in the injection instrument) (the details thereof will be mentioned later).

The IOL 100 in this embodiment is a three-piece IOL produced by separately making the optic part and the support parts and then joining them. Alternatively, the IOL 100 may be a one-piece IOL produced by integrally molding an optic part and support parts of a single material.

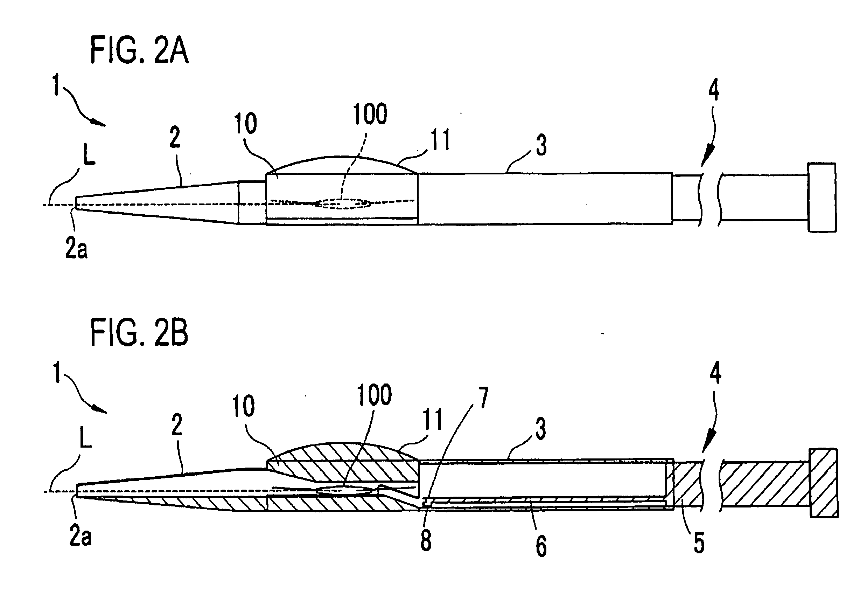

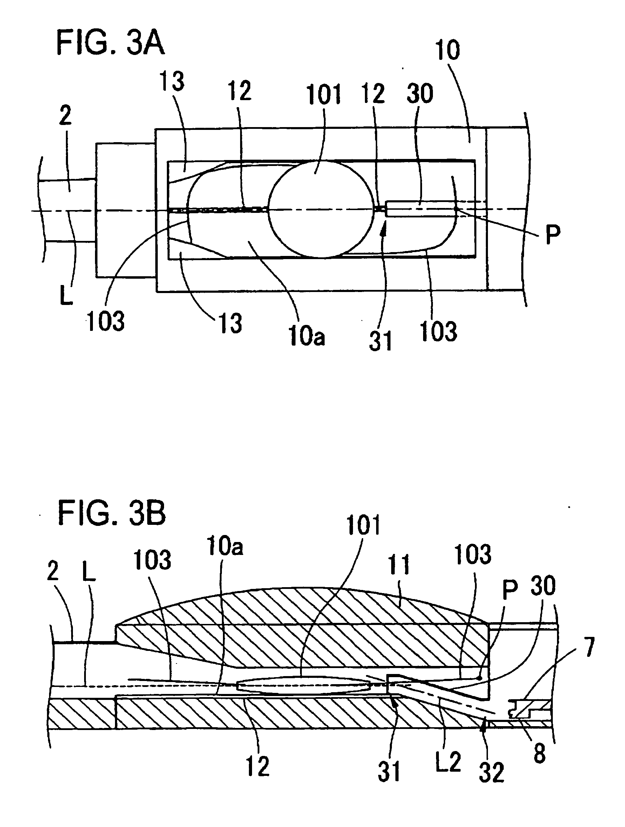

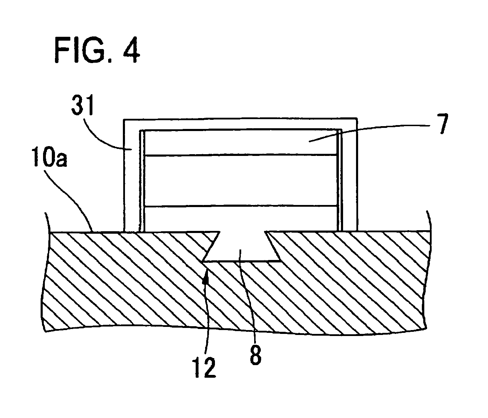

A configuration of the IOL injection instrument 1 in this embodiment will be explained. Figs. 2A and 2B are schematic views to explain the IOL injection instrument 1; Fig. 2A is a schematic side view thereof and Fig. 2B is a schematic sectional view of the same. Figs. 3A and 3B are schematic views to explain a configuration of the setting part 10 and its surrounding; Fig. 3A is a schematic plan view thereof and Fig. 3B is a longitudinal sectional view (a sectional view along the axis L) of the same. In Fig. 3A, for convenience of explanation, a cover 11 has been removed. Fig. 4 is a view to explain a centering part mentioned later. For convenience of explanation, the support parts 103 of the IOL 100 are illustrated on a sectional view (Figs. 2B and 3B).

As shown in Fig. 2A, the IOL injection instrument 1 includes, in the order of insertion into an eye, an injection part 2 to be inserted in an incision made in the eye, the setting part 10 in which the IOL 100 is set, the setting part 10 being provided in a rear end of the injection part 2, an outer cylindrical part 3 that has a cylindrical internal wall surface and is connected to the setting part 10, and a rod-shaped push-out member 4 placed extending through the inside of the outer cylindrical part 3 to push out the IOL 100 through the injection part 2. The cover 11 provided on the setting part 10 is openable and closable. When the cover 11 is opened, the IOL 100 can be put in the setting part 10. Furthermore, the height of an upper wall of the setting part 10 is made as lower as possible but the details are omitted. When the cover 11 is closed, upward movement of the IOL 100 in the setting part 10 is restricted. When the IOL 100 put on a setting surface 10a of the setting part 10 is pushed by the push-out member 4, the IOL 100 is gradually folded (rounded) in the injection part 2 and ejected (injected into the eye) through a front end 2a of the injection part 2. At that time, the IOL 100 is moved in an advancing direction along the axis L. In Fig. 2A, the IOL 100 placed in the setting part 10 is indicated by a dotted line to schematically show a position of the IOL 100.

As shown in Fig. 2B, the injection part 2 is constituted of a hollow member that has a smaller diameter than the diameter of the outer cylindrical part 3 located at the rear thereof and that is tapered toward the front end 2a in order to allow the IOL 100 to be easily injected in a folded state without widening an incision formed in the eye more than necessary. A cross section (a section perpendicular to the axis L) of the injection part 2 is nearly circular. The injection part 2 is provided, on its proximal end side, with side walls 13 to round (fold) the optic part 101 to be pushed out (see Fig. 3A). The side walls 13 are arranged on right and left sides (on upper and lower sides in Fig. 3A) with respect to the optic part 101. A distance between the opposite side walls 13 is determined to be smaller toward the front end 2a. Accordingly, the optic part 101 can be rounded by the side walls 13 as the optic part 101 is moved toward the front end 2a.

The push-out member 4 includes a press part to be pressed by an operator and is constituted of an axial base part 5 for preventing axial deflection or displacement during movement through the inside of the outer cylindrical part 3, a push rod 6 extending forward from a front end of the axial base part 5 and having a smaller diameter than the axial base part 5, and a distal end portion 7 provided at a distal end of the push rod 6 to come into contact with the edge 102 to hold the optic part 101. The push rod 6 is arranged eccentrically with respect to the center axis (concentric with the axis L in this embodiment) of the axial base part 5 and the push rod 6 is placed below the setting surface 10a in this embodiment. The push-out member 4 is attached to be movable forward and backward (advanceable and retractable) in a longitudinal direction (along the axis L) inside the outer cylindrical part 3. The distal end portion 7 is formed with a recess (a guide groove) for catching the optic part 101 in order to hold and push out the optic part 101. The push rod 6 and the distal end portion 7 are made of a relatively soft material so as not to damage the IOL 100 and molded of a material having adequate flexibility with such a degree as to allow the push-out member 4 to move forward and backward while being bent (deformed) along a bending member 30 mentioned later, for example, resin such as polypropylene. The length of the push rod 6 is determined to be long enough to make the distal end portion 7 sufficiently protrude out of the injection part 2 when the push-out member 4 is pushed into the outer cylindrical part 3. The push rod 6 is positioned on an axis different from the movement axis L of the IOL until the push-out operation of the IOL 100 is pushed. The push rod 6 is bent by passing through the bending member 30 mentioned later when the push-out operation is started by the push-out member 4. The push rod 6 positioned on the axis different from the movement axis L is bent before the push rod 6 comes into contact with the optic part 101 in process of the push-out operation. Thus, the distal end portion 7 comes to coincide with the movement axis L of the IOL 100. By forward and backward movement of the push rod 6 on the movement axis L, the IOL 100 is pushed out along the movement axis L. A lower side of the distal end portion 7 is formed with a protrusion 8 (the details thereof will be mentioned later).

The setting part 10 is formed with the setting surface 10a on which the optic part 101 of the IOL 100 is to be set. A distance between right and left side walls of the setting part 10 is determined to be slightly smaller than the diameter of the optic part 101 and not to interfere with the support parts 103. The side walls of the setting part 10 with which the edge 102 of the optic part 101 comes into contact are formed in a recessed shape for receiving the outer periphery of the optic part 101 in order to fixedly hold the optic part 101 without applying stress thereto. The setting part 10 is provided with the bending member (a guide member) 30 arranged to pass the push rod 6 therethrough and guide movement of the push rod 6. The bending member 30 serves to bend (deform) the push rod 6 and then move it along the movement axis L. The bending member 30 is a tubular member being bent relative to the movement axis L and extending by a predetermined length. The bending member 30 is designed to have an inner diameter equal to or slightly larger than the diameter of the distal end portion 7. A central axis L2 of the tubular bending member 30 is formed to be oblique with respect to the setting surface 10a. The bending member 30 is provided to intersect with the setting surface 10a at a predetermined angle. The bending member 30 has an exit 31 at one end of a front side (a side closer to the IOL 100), through which the distal end portion 7 is fed out from the bending member 30, and an entrance 32 at the other end (a rear end), through which the distal end portion 7 is inserted into the bending member 30. The exit 31 is formed as high as the IOL 100 (the optic part 101) (the exit 31 is positioned on the movement axis L) so that the distal end portion 7 having exited from the exit 31 is moved along the movement axis L. Furthermore, the inside of the tube (an inner passage of the bending member 30) near the exit 31 is formed in a shape along the movement axis L by a predetermined length. The exit 31 is arranged so as to be positioned between the optic part 101 placed on the setting surface 10a and the rear support part 103. The setting surface 10a is formed with a groove 12 along the movement axis L (the details will be mentioned later).

A bent portion of the bending member 30 is formed in a shape that will not come into contact with the rear support part 103 in a state with no stress applied when the exit 31 is positioned between the optic part 101 and the rear support part 103. Herein, the bending member 30 has a shape extending obliquely downward to the rear side. Herein, the "contact" state with stress applied means a state in which the rear support part 103 is constantly in contact with the bending member 30 to such a degree that the rear support part 103 is deformed, but does not include a state in which the rear support part 103 is in contact with the bending member 30 to such a degree that the rear support part 103 is not deformed. Accordingly, an inclination angle of the bending member 30 relative to the setting surface 10a is determined at an angle allowing an outer wall of the tubular member (the bending member 30) extending downward to the rear side from the exit 31 to avoid the rear support part 103 without contacting therewith and also an inclination angle at which the push rod 6 can be bent smoothly in process of moving forward and backward and then pushing out the IOL.

The exit 31 is preferably placed in a position as close to the optic part 101 as possible. The closer to the optic part 101 the exit 31 is, the above condition can be satisfied even if the central axis L2 of the bending member 30 and the axis L become close to parallel. This makes it possible to reduce a load on the push rod 6 to be bent by passing through the bending member 30.

A following explanation will be given to centering for making the forward and backward moving direction of the distal end portion 7 (the push rod 6) approximately coincide with the movement axis (the axis L) of the optic part 101 and also preventing axial deflection during forward and backward movement of the push rod 6 and the distal end portion 7. Fig. 4 is a cross sectional view (a sectional view perpendicular to the axis L) of the setting part 10. For convenience of explanation, the distal end portion 7 and the setting surface 10a are mainly shown.

The protrusion 8 provided on the lower side of the distal end portion 7 is engaged in the groove 12 formed in the setting surface 10a so that the protrusion 8 is movable forward and backward with respect to the setting surface 10a. This configuration allows the centering of the push rod 6. As illustrated, the groove 12 is a dovetail groove having a nearly trapezoidal shape in cross section, which becomes narrower upward between opposite wall surfaces. The protrusion 8 has a nearly trapezoidal shape in cross section so as to engage in the groove 12. A front end face of the protrusion 8 is formed with an oblique cutout for facilitating engagement with the groove 12.

By such configuration, the protrusion 8 engaging in the groove 12 is unlikely to disengage from the groove 12. The groove 12 is formed in straight shape along the movement axis L from the exit 31 toward the injection part 2 (see Fig. 3A). The protrusion 8 has a nearly trapezoidal cross section for engagement with the groove 12 so that the nearly trapezoidal cross section continues in the forward and backward direction to ensure the length enough to prevent deflection of the distal end portion 7 when the protrusion 8 is engaged in the groove 12.

The protrusion 8 becomes engaged in the groove 12 when the distal end portion 7 is moved out from the exit 31 by the push-out operation of the IOL 100. Thus, the distal end portion 7 is prevented from deflecting up and down, and right and left, relative to the setting surface 10a and allowed to move in the forward and backward direction (a direction along the axis L), that is, the movement axis of the optic part 101 and the forward and backward moving direction of the distal end portion 7 are made to substantially coincide with each other. Thus, the IOL 100 will be pushed out appropriately by the distal end portion 7 (the push rod 6).

In the above embodiment, the centering is achieved by using the dovetail groove but it is not limited thereto. Any configuration capable of preventing axial deflection of the distal end portion 7 (i.e., capable of making the movement axis of the optic part and the forward and backward moving direction of the distal end portion 7 substantially coincide) may be adopted. For instance, a groove formed in the forward and backward direction may be provided with a stopper structure for preventing upward slippage of the distal end portion 7. Another configuration may be provided by using a groove having an inverted T-shaped cross section and a protrusion having an inverted T-shaped cross section engageable in the groove. Yet another configuration may be provided by forming a slit in the setting surface 10a and a protrusion on the lower side of the distal end portion 7 so as to be engageable in the slit. This makes it possible to prevent axial deflection of the distal end portion 7 in a right and left direction and a downward direction. Furthermore, the protrusion may be formed to be long to some extent in the forward and backward direction, so that the protrusion is less likely to disengage from the groove and upward axial deflection of the distal end portion 7 can be restrained.

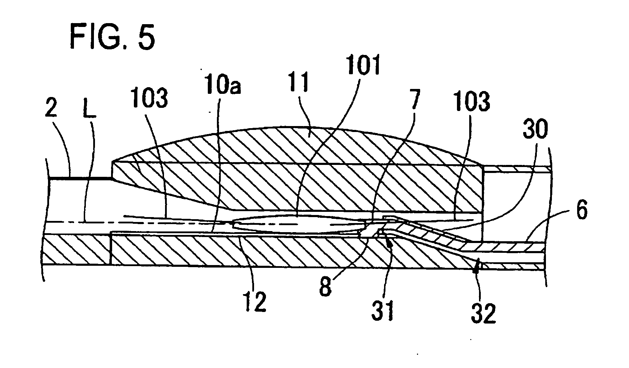

In the IOL injection instrument 1 having the above configuration, the push-out operation of the IOL 100 will be explained below. Fig. 5 is a sectional view showing a state where the push rod 6 is pushed forward from the state of Fig. 3B to protrude from the exit 31 until the distal end portion 7 reaches the IOL 100.

The operator uses the IOL injection instrument 1 by opening the cover 11 and putting the IOL 100 on the setting surface 10a. Alternatively, the operator uses an IOL injection instrument shipped from a factory after the optic part 100 is set in advance on the setting surface 10a at the factory. When the press part of the axial base part 5 is pressed by the operator, the push rod 6 advances along the axis L. When further pressed, the distal end portion 7 is charged (inserted) in the entrance 32, the push rod 6 is bent upward along an internal shape of the bending member 30 to move along the central axis L2. In this way, the push rod 6 and the distal end portion 7 are moved from below at a certain angle with respect to the setting surface 10a. The distal end portion 7 having reached the exit 31 is moved out from the exit 31 by further pressing, and then the protrusion 8 of the distal end portion 7 engages in the groove 12. By this engagement between the protrusion 8 and the groove 12, the distal end portion 7 can be pushed forward while the axial deflection thereof is restrained (the axis L and the forward and backward direction of the distal end portion 7 are made to substantially coincide). Accordingly, as the push rod 6 positioned in the bending member 30 is gradually pushed or protruded out, the distal end portion 7 holds the optic part 101 to push the IOL 100 into the injection part 2.

At that time, the protrusion 8 is engaged in the groove 12 before the distal end portion 7 comes into contact with the optic part 101. The IOL 100 is therefore appropriately pushed forward. When further pushed forward, the IOL 100 is rounded by the side walls 13 and, in a folded state, is pushed out of the injection part 2 through the front end 2a. In the above operation, the support part 103 is moved together with the optic part 101 without being caught between the optic part 101 and the distal end portion 7, and then the support part 103 is unbent by the side walls 13 and the injection part 2 and pushed out through the front end 2a.

In the present embodiment explained above, the guide member is constituted of the cylindrical bending member (a tubular member) 30 having the exit 31 and the entrance 32 but it is not limited thereto. The guide member has only to be configured to guide the forward and backward direction of the push rod 6 from a direction (herein, from below) different from the movement axis L to the same axis as the movement axis L (configured to bend the push rod and then guide it to move along the movement axis). For instance, as the guide member, a rear portion of the setting part 10a is formed to slant downward and a groove (a guide groove formed on the rear portion of the setting surface 10a to extend downward) engageable with the protrusion 8 of the distal end portion 7 is formed along the movement axis L on the slant surface. This groove is formed with the same shape as the above groove 12 and continuous from a rear end of the groove 12. In the push-out operation, when the distal end portion 7 (the protrusion 8) is moved forward and backward while engaging in the groove of the slant surface, the push rod 6 is moved along the slant surface and at a predetermined angle with respect to the movement axis L. When the distal end portion 7 coincides with the movement axis L, as in the above case, the protrusion 8 is engaged in the groove 12 and the push rod 6 (the distal end portion 7) is centered to push the IOL 100. The groove formed in the slant surface does not have to be formed linearly on the slant surface along the movement axis L. For instance, the length of the groove (the length toward the rear side) may be short if only it is long enough to make the forward and backward moving direction of the push rod 6 coincide with the movement axis L when the distal end portion 7 reaches the setting surface 10a.

## Claims

1. An intraocular lens injection instrument (1) comprising:
an injection part (2) for injecting an intraocular lens (IOL) (100) into an eye through an incision to be formed in the eye, the IOL having a deformable optic part (101) and a pair of support parts (103) each having a loop shape extending from the optic part, and the injection part having a tapered internal shape that allows the IOL to be folded;
a setting part (10) connected to a rear end of the injection part and configured to set therein the IOL in a state with no stress applied;
a main unit (3) of a cylindrical configuration having the injection part and the setting part at a front end; and
a push-out member (4) having a push rod (6) provided to be movable forward and backward in a direction of a movement axis (L) in the main unit in order to push the IOL along the movement axis (L) out of a front end of the injection part,
**characterized in that** the IOL injection instrument further comprises:
a guide member (30) provided in a rear of the setting part and arranged to guide forward and backward movement of the push rod, the guide member including an exit (31) for feeding the push rod and located in a position on the movement axis between the optic part and a rear one of the support parts and an entrance (32) for receiving the push rod and located in a position rearward than the exit and out of the movement axis to prevent the rear support part from becoming deformed, and
the push rod has flexibility to be deformed along the guide member.

2. The IOL injection instrument according to claim 1, wherein
the guide member is formed with a first guide groove (12), and the push rod is provided with a protrusion (8) engageable with the first guide groove.

3. The IOL injection instrument according to claim 2, wherein the first guide groove is formed to extend from the entrance toward the exit.

4. The IOL injection instrument according to claim 2 or 3, wherein
the setting part and the injection part are provided with a second guide groove (12) continuous with the first guide groove.

5. The IOL injection instrument according to claim 4, wherein the first and second guide grooves are formed to extend from the exit toward the injection part.

## Patentansprüche

1. Injektionsinstrument für eine Intraokularlinse (1), das umfasst:
einen Injektionsteil (2) zum Injizieren einer Intraokularlinse (IOL) (100) in ein Auge durch einen Einschnitt, der in dem Auge gemacht werden soll, wobei die IOL einen verformbaren optischen Teil (101) sowie ein Paar Stützteile (103) aufweist, die jeweils eine Schlaufenform aufweisen, die sich vom optischen Teil aus erstrecken und wobei der Injektionsteil eine konische Innenkontur aufweist, die es der IOL ermöglicht, umgebogen zu werden;
einen Einsetzteil (10), der mit einem hinteren Ende des Injektionsteils verbunden und so eingestellt ist, um die IOL darin in einem Zustand einzusetzen, in dem keine Beanspruchung auf sie wirkt;
eine Haupteinheit (3) mit einer zylinderförmigen Ausgestaltung, welche den Injektionsteil und den Einsetzteil an einem vorderen Ende aufweist; und
ein Ausschiebeelement (4), das eine Ausschiebestange (6) aufweist, die in der Haupteinheit in einer Richtung der Bewegungsachse (L) vor und zurück bewegbar ist, um die IOL entlang der Bewegungsachse (L) aus einem vorderen Ende des Injektionsteils auszuschieben,
**dadurch gekennzeichnet, dass** das IOL-Injektionsinstrument ferner umfasst:
ein Führungselement (30), das in einem hinteren Bereich des Einsetzteils vorgesehen ist und angeordnet ist, um eine Vorwärts- und Rückwärtsbewegung der Ausschiebestange zu führen, wobei das Führungselement einen Ausgang (31) zum Vorschieben der Ausschiebestange enthält, der sich in einer Lage auf der Bewegungsachse zwischen dem optischen Teil und dem hinteren der Stützteile befindet, und einen Eingang (32) zum Empfangen der Ausschiebestange, der sich in einer Lage weiter hinten als der Ausgang und außerhalb der Bewegungsachse befindet, um den hinteren Stützteil davor zu schützen, verformt zu werden, und
wobei die Ausschiebestange eine Flexibilität aufweist, um entlang des Führungselements verformt zu werden.

2. IOL-Injektionsinstrument nach Anspruch 1, wobei
das Führungselement mit einer ersten Führungsnut (12) ausgebildet ist und die Ausschiebestange mit einem Vorsprung (8) vorgesehen ist, der mit der ersten Führungsnut in Eingriff bringbar ist.

3. IOL-Injektionsinstrument nach Anspruch 2, wobei die erste Führungsnut so gebildet ist, dass sie sich vom Eingang in Richtung des Ausgangs erstreckt.

4. IOL-Injektionsinstrument nach Anspruch 2 oder 3, wobei
der Injektionsteil und der Einsetzteil mit einer zweiten Führungsnut (12) vorgesehen sind, welche mit der ersten Führungsnut zusammenhängt.

5. IOL-Injektionsinstrument nach Anspruch 4, wobei die erste und die zweite Führungsnut so gebildet sind, dass sie sich vom Ausgang in Richtung des Injektionsteils erstrecken.

## Revendications

1. Instrument d'injection de lentille intraoculaire (1) comprenant :
une partie d'injection (2) pour injecter une lentille intraoculaire (IOL) (100) dans un oeil par une incision destinée à être formée dans l'oeil, la IOL ayant une partie optique déformable (101) et une paire de parties de support (103) ayant chacune une forme de boucle s'étendant à partir de la partie optique, et la partie d'injection ayant une forme interne progressivement rétrécie qui permet à la IOL d'être pliée ;
une partie d'ajustement (10) raccordée à une extrémité arrière de la partie d'injection et configurée pour placer à l'intérieur de cette dernière la IOL dans un état sans tension appliquée ;
une unité principale (3) d'une configuration cylindrique ayant la partie d'injection et la partie d'ajustement au niveau d'une extrémité avant ; et
un élément de poussée (4) ayant une tige de poussée (6) prévue pour être mobile vers l'avant et vers l'arrière dans une direction d'un axe de déplacement (L) dans l'unité principale afin de pousser la IOL le long de l'axe de déplacement (L) à l'extérieur d'une extrémité avant de la partie d'injection,
**caractérisé en ce que** l'instrument d'injection de IOL comprend en outre :
un élément de guidage (30) prévu dans une partie arrière de la partie d'ajustement et agencé pour guider le mouvement vers l'avant et vers l'arrière de la tige de poussée, l'élément de guidage comprenant une sortie (31) pour amener la tige de poussée et positionnée dans une position sur l'axe de déplacement entre la partie optique et une partie arrière des parties de support et une entrée (32) pour recevoir la tige de poussée et positionnée dans une position plus vers l'arrière que la sortie et hors de l'axe de déplacement pour empêcher la déformation de la partie de support arrière, et
la tige de poussée présente une flexibilité destinée à être déformée le long de l'élément de guidage.

2. Instrument d'injection de IOL selon la revendication 1, dans lequel :
l'élément de guidage est formé avec une première rainure de guidage (12), et la tige de poussée est prévue avec une saillie (8) pouvant se mettre en prise avec la première rainure de guidage.

3. Instrument d'injection de IOL selon la revendication 2, dans lequel la première rainure de guidage est formée pour s'étendre à partir de l'entrée vers la sortie.

4. Instrument d'injection de IOL selon la revendication 2 ou 3, dans lequel :
la partie d'ajustement et la partie d'injection sont prévues avec une seconde rainure de guidage (12) continue avec la première rainure de guidage.

5. Instrument d'injection de IOL selon la revendication 4, dans lequel les première et seconde rainures de guidage sont formées pour s'étendre à partir de la sortie vers la partie d'injection.
